# EUROPEAN PATENT APPLICATION

(11) **EP 3 206 147 A1**
(43) Date of publication of application: **16.08.2017**
(21) Application number: 17155224.3
(22) Date of filing: 08.02.2017
(51) Int. Cl.: G06F 19/00, H04B 10/116

(54) **VISUAL COMMUNICATION BETWEEN MOBILE COMMUNICATION DEVICES AND DRUG DELIVERY DEVICES**

(30) Priority: 11.02.2016 US 201615041655
(71) Applicant: West Pharmaceutical Services, Inc., Exton, PA 19341 (US)
(72) Inventor: ESPOSITO, Anthony G., Fountain Hill, AZ, 85268 (US)
(74) Representative: Lippert Stachow Patentanwälte Rechtsanwälte

(57) **Abstract**

Method and apparatus are directed to a visible light communication module configured to transmit information associated with a drug delivery device. The visible light communication module comprises a controller and a light source. The controller is configured to: receive
information associated with the drug delivery device; and modulate, in accordance with the received
information, at least one drive signal for driving the light source configured to emit visible light signals of a first wavelength. The light source is configured to emit the visible light signals in accordance with the modulated at least one drive signal.

## Description

### BACKGROUND OF THE INVENTION

The present invention relates to methods and apparatus for visual communication, and, more particularly, visual communication between a mobile communication device and a drug delivery device.

A variety of medical conditions exist that require regular treatment by drug delivery, such as an injection. These injections can be performed by drug injection devices (e.g., needle injection devices), which can be operated by various individuals including medical personnel and patients themselves. As an example, type-1 and type-2 diabetes can be treated by patients themselves by injection nf Insulin (loses, for example, once or several times per day. Increased portability and ease of use of such drug injection devices have enabled patients to administer a self-regulated medical treatment regime which in turn provides an increased level of patient autonomy and privacy.

One of the main concerns, however, of drug delivery devices is the patient's compliance with the intended usage. For example, by not following a prescribed drug regime or using the device incorrectly, the efficacy of a prescribed drug may be decreased. Moreover, improper usage can be detrimental to the patient's health, as improper use of a drug delivery device can lead to drug complications. As such, it would be beneficial for drug delivery device information including usage information to be made easily available to health care personnel for analysis.

Accordingly, there is a need for an apparatus to track and transmit usage information of a drug delivery device to health care personnel to ensure that the device is being used properly as well as according to the prescribed regimen.

### BRIEF SUMMARY OF THE INVENTION

Briefly stated, one embodiment of the present invention is a method for transmitting information associated with a drug delivery device. The method comprises receiving information associated with the drug delivery device; modulating, in accordance with the received information, at least one drive signal for driving at least one light source configured to emit visible light signals of a first wavelength; and emitting the visible light signals in accordance with the modulated at least one drive signal.

Another embodiment of the present invention is directed to a visible communications module configured to transmit information associated with a drug delivery device. The visible communications module comprises a controller and a light source. The controller is configured to: receive information associated with the drug delivery device; and modulate, in accordance with the received information, at least one drive signal for driving the light source configured to emit visible light signals of a first wavelength. The light source is configured to emit the visible light signals in accordance with the modulated at least one drive signal.

### BRIEF DESCRIPTION OF THE SEVERAL VIEWS OF THE DRAWINGS

The following detailed description of embodiments of the invention will be better understood when read in conjunction with the appended drawings. For the purpose of illustrating the invention, there are shown in the drawings embodiments which are presently preferred. It should be understood, however, that the invention is not limited to the precise arrangements and instrumentalities shown.

In the drawings:
Fig. 1 is an example diagram illustrating communication between a drug delivery device and a computing device according to an embodiment of the present invention;
Fig. 2 is a schematic block diagram illustrating components of a visible communications module including one light source according to an embodiment of the present invention;
Fig. 3 is a schematic block diagram illustrating components of another visible communications module including three light sources according to an embodiment of the present invention;
Fig. 4 is a schematic block diagram illustrating an example of use of drug delivery device information received by the computing device from a drug delivery device according to an embodiment of the present invention; and
Fig. 5 is a flow diagram of one embodiment of a method for transmitting drug delivery device information from a drug delivery device to a computing device.

### DETAILED DESCRIPTION OF THE INVENTION

Certain terminology is used in the following description for convenience only and is not limiting. The words "lower," "bottom," "upper" and "top" designate directions in the drawings to which reference is made. Unless specifically set forth herein, the terms "a," "an" and "the" are not limited to one element, but instead should be read as meaning "at least one." The terminology includes the words noted above, derivatives thereof and words of similar import. It should also be understood that the terms "about," "approximately," "generally,"
"substantially" and like telins, used herein when referring to a dimension or characteristic of a component of the invention, indicate that the described dimension/characteristic is not a strict boundary or parameter and does not exclude minor variations therefrom that are functionally similar.
At a minimum, such references that include a numerical parameter would include variations that, using mathematical and industrial principles accepted in the art (e.g., rounding,
measurement or other systematic errors, manufacturing tolerances, etc.), would not vary the least significant digit.

Disclosed embodiments of the present invention provide a system for communicating information regarding the Operation of a drug delivery device to a computing device. The information may include dose information, drug delivery device identification information, drug delivery device usage information, and any other information associated with the drug delivery device. The computing device can then transmit drug delivery device information to a health care provider, clinic, doctor, or the like, so that such drug delivery device information can be analyzed by a health care professional to improve the patient's habits, make recommendations for device use, etc.

In the present embodiment, the drug delivery device transmits information to the computing device through visible light communication. More specifically, the drug delivery device communicates with the computing device using variations of visible light (color, intensity, on/off duty cycle, position or the like) to encode and transmit information. The variations of visible light can be detected by a photosensitive device, such as, for example, an image sensor, such as a photodiode of the computing device, and can be restored or decoded back into the transmitted information.

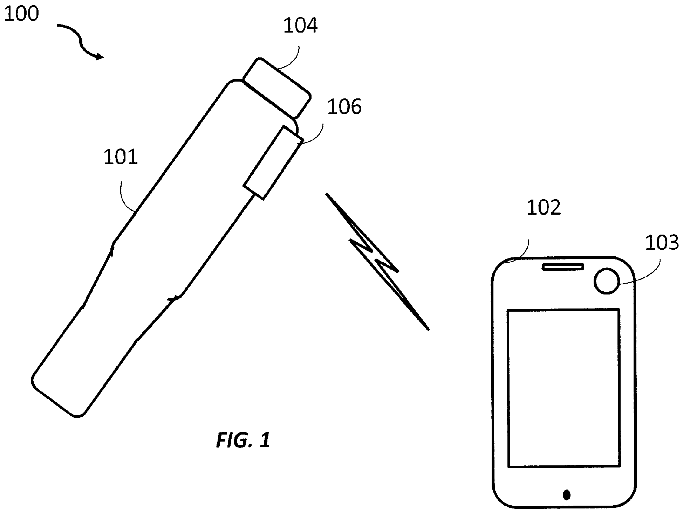

As shown in Fig. 1, a drug delivery device 100 is in communication with a computing device 102. More importantly, the computing device 102 is configured to receive data from the drug delivery device 100. The drug delivery device 100 may include one or more containers (not shown) for holding the medicament. The drug delivery device 100 may take the form of a drug delivery device disclosed in U.S. Patent No. 6,387,078 to Gillespie, III, the disclosure of which is hereby incorporated herein by reference in its entirety. It should be noted, however, that it is within the intent and scope of the present invention that any drug delivery device can be used.

The drug delivery device 100 comprises a housing 101, an actuator 104, and a visible light communication module 106. The housing 101 may take any form or include additional components to facilitate temporary or permanent attachment to the drug delivery device 100. For example, the housing 101 may optionally include built in connectors and/or fasteners (for example snaps, latches, catches, hooks, clasps and the like) for attachment to the drug delivery device 100. The housing 101 is preferably formed of a generally rigid, preferably polymeric material, such as polyvinyl chloride or some other such polymeric material well-known to those of ordinary skill in the art.

The actuator, in the present embodiment a button 104, is configured to initiate Operation of the drug delivery device 100 upon depression. The visible light communication module 106 is configured to communicate information associated with the drug delivery device 100 to the computing device 102. It should be noted that the spatial position of the visible light communication module 106 on the drug delivery device 100 is for illustrative purposes only, and may be attached anywhere on or within the drug delivery device 100 suitable for transmission of drug delivery device information via visible light communication.

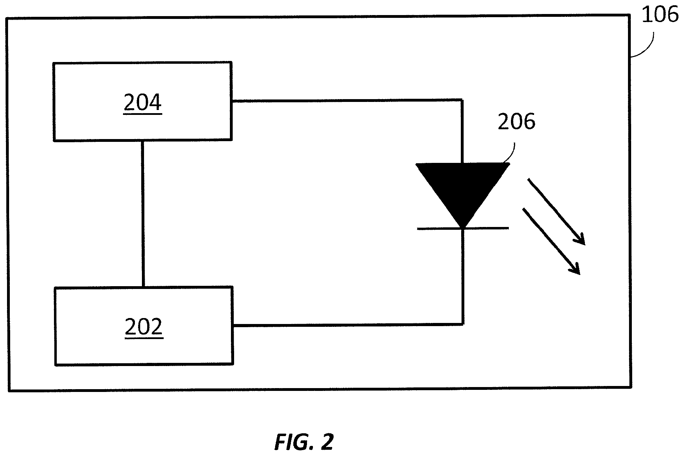

A patient may use the drug delivery device 100 to inject a dose of medication into his or herself. Such an injection begins upon actuation of the drug delivery device 100, such as, for example, through depression of the actuation button 104. Upon depression of the actuation button 104, a seal on a power source (shown in Fig. 2) may be removed, thereby providing power to the visible light communication module 106. The visible light communication module 106 is configured to transmit information associated with the drug delivery device 100 to the computing device 102 once provided with power from the power source. Such information may include but is not limited to dose information, drug delivery device identification information (e.g., a serial number) drug delivery device usage information, and any other information associated with the drug delivery device.

The computing device 102 may refer to a smart phone, tablet, e-reader, iPad, mobile gaming console, personal computer, mp3 player, iPod or any other device which includes an image sensor 103 capable of receiving and recovering a visible light communication signal.

Fig. 2 is a schematic block diagram showing components of a first embodiment of the visible light communication module 106. The visible light communication module 106 comprises a power source 202, a microcontroller 204, and a light source 206 which may be configured to Illuminate in any desired color. The power source 202 is configured to provide energy for the Operation of the visible light communication module 106 upon activation of the drug delivery device 100. The power source 202 is preferably a metal air battery, such as a zinc-air battery. Alternatively, the power source 202 may use lithium-ion or nickel-metal hydride technology or the like. The power source 202 may include a non-rechargeable battery
employing alkaline or zinc-carbon technology or the like. In light of the embodiments discussed herein, it should be appreciated that other types of battery technologies as known in the art may be used as well.

The drug delivery device 100 transmits drug delivery information using variations of visible light (color, intensity, on/off duty cycle, or position) to transmit information. To do this,
the microcontroller 204 can receive information associated with the drug delivery device 100,
such as via, one or more sensors (not shown) of the drug delivery device 100. Such information may take many forms, such as a digital signal. Based on this information (e.g., the digital signal), the microcontroller 204 is configured to cause the light source 206 to blink (on/off) in a particular sequence or frequency. More specifically, the microcontroller 204 is configured to modulate a drive signal to drive the light source, such as, for example, via On-Off Keying (00K). With such a modulation scheme, the microcontroller 204 is configured to blink the light source on and off (e.g., switching between "1" and "0") on the basis of information to be transmitted. For example, the data bit "0" and "1" can be transmitted by choosing two separate levels of light intensity. For example, the microcontroller may cause the light source to switch on and off in a sequence in a particular duration of time. Such blinking may represent an encoding of data associated with the drug delivery device to which the visible light communication module 106 is coupled. It should be noted that other modulation techniques may be employed and light source variations may be employed. For example, phases and intensities of light signal emission may be employed in keeping with the invention. The light source 206 may be any type of light source emitting visible light signals, for example, a light emitting diode (LED), a fluorescent, or incandescent lamp or the like.

The blinking signal can be detected by the computing device 102, such as, for example, by the image sensor 103, such as a photodiode, and can be decoded or restored back into the transmitted information. Such information may then be re-transmitted via any known communication technique to a healthcare provider. ft is known that the computing device 102,
such as, for example, a smartphone, includes the image sensor 103 having a capture rate of approximately 60 frames per second, allowing for a data transfer of approximately 30 captures per second. As such, a transmission of 8 bytes of data would require approximately 2.13 seconds.

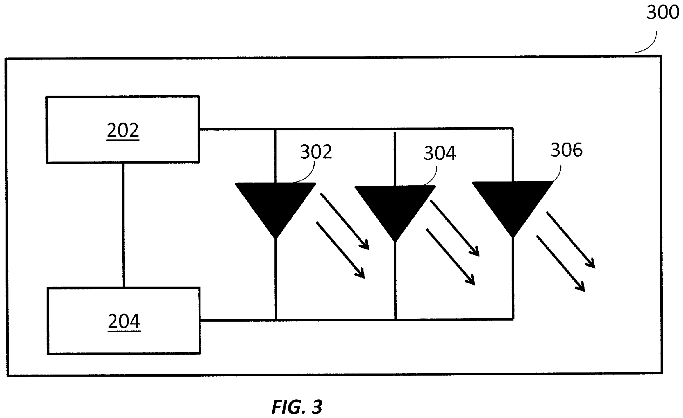

It should be noted that the number of light sources is not limited to one, as according to embodiments of the present invention, a visible light communication module may include any number of light sources. For example, as shown in Fig. 3, a visible light communication module 300 includes three light sources 302, 304, 306. Each of the light sources 302, 304, 306 may be configured to emit visible light signals with different colors having different wavelengths, such as red light having a wavelength of 640-780nm, green light having a wavelength of 505-525nm,
or a blue light having a wavelength of 470-505nm. Therefore, as compared to the light
transmission having only two choices of transmitting or not transmitting light representing "1" or "0", which can only transmit data of 1 bit during a unit of time, the visible light communication module 300 is capable of transmitting more bits of data per unit of time, or the same amount of data in a shorter period of time. For example, in a single light source implementation, such as
described in connection with Fig. 2 above, a transmission of 8 bytes of data would require approximately 2.13 seconds. However, with three light sources, such as each configured to emit a different color, a transmission of 8 bytes of data would require approximately .71 seconds.

ft should be appreciated by those of ordinary skill in the art that the various electrical/electronic components and the functions presented in Figs. 2 and 3, are merely two illustrations of the electrical/electronic workings of embodiments of the present invention. Thus,
it should be clearly understood that other components may be substituted for any of the components shown in Figs 2 and 3, and that components which perform other functions may alternatively be employed. In other words, the present invention is not limited to the precise structure and Operation of the electrical/electronic and related components shown in Figs. 2 and 3

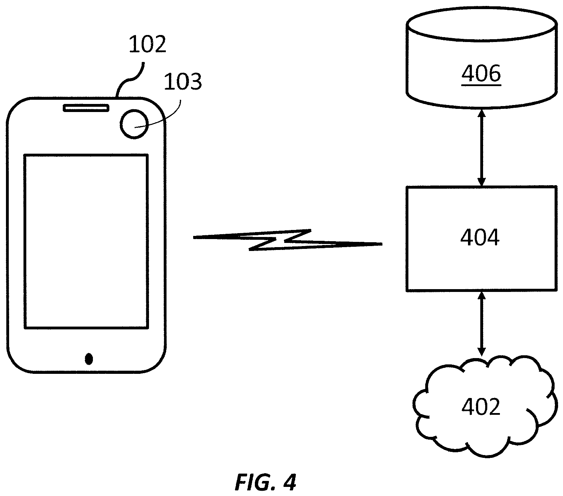

Fig. 4 illustrates an example of the use of the drug delivery device information
received by the computing device 102, such as via visible light communications from the visible light communication module 106, 300 of the drug delivery device 100. The computing device 102 may connect to a network 402, which may be, for example, a wired or wireless network. Although the network 402 may be a single network, the network 402 may be representative of
two or more networks that allow the computing device 102 to communicate with a networked server 404. The network 402 may be embodied as one or more of the Internet, a wireless network, a wired network, a cellular network, or a Fiber optic network. In other words, the network 402 may be any data communication protocol or protocols that facilitate data transfer between two or more devices. The networked server 404 may also connect to storage 406 for storing data or retrieving data corresponding to drug delivery device information (e.g., converted by the computing device to digital data). The networked server 404 may include one or more servers, desktop computers, mainframes, minicomputers, or other computing devices capable of executing computer instructions and storing data. In some examples, functions attributable to the computing device 102 may be attributed to respective different servers such as networked server
404 for respective functions. The storage 406 may include one or more memories, repositories,
hard disks, or any other data storage device. In some examples, the storage 406 may be included within the networked server 404.

The storage 406 may be included in, or described as, cloud storage. The networked server 404 may access the cloud and retrieve data as necessary. In some examples, the storage 406 may include relational database management system (RDBMS) software. In one example,
the storage 406 may be a relational database an accessed using a Structured Query Language (SQL) interface that is well known in the art. The storage 406 may alternatively be a separate networked computing device and accessed by the networked server 404 through a network interface or system bus. The storage 406 may in other examples be an Object Database Management System (ODBMS), Online Analytical Processing (OLAP) database or other suitable data management system.

The drug delivery device information may be relayed to doctors, clinicians, and/or other health care providers via the network 402 or via short messaging service (SMS) text data. Alternatively, doctors, clinicians, and/or other health care providers may access the drug delivery information from the storage 406.

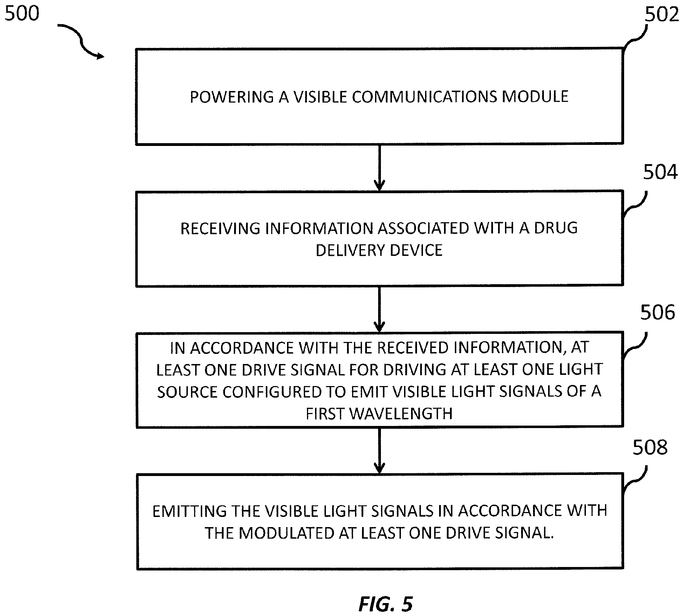

Fig. 5 is a flow diagram 500 of one embodiment of a method for transmitting drug delivery device information to a computing device. The method may comprise a number of steps which may be performed in any suitable order. Step 502 comprises providing power to the visible communications module 106. Such power may be provided in response to actuation of the drug delivery device 100. Step 504 comprises receiving information associated with the drug
delivery device 100. Step 506 comprises modulating (or encoding), in accordance with the received information, at least one drive signal for driving at least one light source configured to emit visible light signals of a first wavelength. Step 508 comprises emitting the visible light signals in accordance with the modulated (or encoded) at least one drive signal.

It will be appreciated by those skilled in the art that changes could be made to the embodiments described above without departing from the broad inventive concept thereof. It is understood, therefore, that this disclosure is not limited to the particular embodiments disclosed, but it is intended to cover modifications within the spirit and scope of the appended claims.

## Claims

1. A method for transmitting information associated with a drug delivery device, the method comprising:
receiving information associated with the drug delivery device;
modulating, in accordance with the received information, at least one drive signal for driving at least one light source configured to emit visible light signals of a first wavelength; and
emitting the visible light signals in accordance with the modulated at least one drive signal.

2. The method of claim 1, wherein the emitting further comprises transmitting the visible light signals to a computing device including an image sensor.

3. The method of claim 1 or 2, wherein the computing device is configured to convert the transmitted visible light signals into digital data associated with the drug delivery device.

4. The method of claims 1 to 3, wherein the at least one drive signal includes at least one drive voltage signal or at least one drive current signal.

5. The method of claims 1 to 4, wherein the received information comprises at least one of an identifier of the drug delivery device, a mode of operation of the drug delivery device, or an ambient temperature of the drug delivery device.

6. The method of claims 1 to 5, wherein the modulating comprises on-off keying.

7. The method of claims 1 to 6, wherein the modulating comprises modulating, in accordance with the received information, at least one drive signal for driving at least one light source configured to emit visible light signals of the first wavelength having the levels of the at least one light source changed between a peak level and a bottom level with different frequencies, different phases, or different amplitudes.

8. A visible communications module configured to transmit information associated with a drug delivery device, the visible communications module comprising:
a controller configured to:
receive information associated with the drug delivery device; and
modulate, in accordance with the received information, at least one drive signal for driving at least one light source configured to emit visible light signals of a first wavelength; and
at least one light source configured to emit the visible light signals in accordance with the modulated at least one drive signal.

9. The visible communications module of claim 8, wherein the at least one light source is further configured to emit the visible light signals to a computing device including an image sensor.

10. The visible communications module of claim 8 or 9, wherein the at least one drive signal includes at least one drive voltage signal or at least one drive current signal.

11. The visible communications module of claims 8 to 10, wherein the received information comprises at least one of an identifier of the drug delivery device, a mode of operation of the drug delivery device, or an ambient temperature of the drug delivery device.

12. The visible communications module of claims 8 to 11, wherein the controller is further configured to modulate the at least one drive signal via on-off keying.

13. The visible communications module of claims 8 to 12, further comprising a power source configured to provide power to the visible communications module.

14. The visible communications module of claim 13, wherein the power source is configured to provide power to the visible communications module in response to activation of the drug delivery device.

15. The visible communications module of claims 8to 14, wherein the at least one light source includes a light emitting diode (LED).
